# EUROPEAN PATENT APPLICATION

(11) **EP 2 184 287 A1**
(43) Date of publication of application: **12.05.2010**
(21) Application number: 09174738.6
(22) Date of filing: 02.11.2009
(51) Int. Cl.: C07F 3/00, C23C 16/18

(54) **Group 2 metal precursors for deposition of group 2 metal oxide films**

(30) Priority: 07.11.2008 US 266806
(71) Applicant: Air Products and Chemicals, Inc., Allentown, PA 18195-1501 (US)
(72) Inventor: Lei, Xinjian, Vista, CA 92081 (US); Spence, Daniel P., Carlsbad, CA 92008 (US); Norman, John Anthony Thomas, Encinitas, CA 92024 (US); Ulman, Michael, Mertztown, PA 19539 (US)
(74) Representative: Muir, Benjamin M. J.

(57) **Abstract**

This invention is related to Group 2 metal-containing polydentate β-ketoiminate precursors and compositions comprising Group 2 metal-containing polydentate β-ketoiminate precursors, wherein the polydentate β-ketoiminate precursors incorporate an alkoxy group in the imino portion of the molecule. The compounds and compositions are useful for fabricating metal containing films on substrates such as silicon, metal nitride, metal oxide and other metal layers via chemical vapor deposition (CVD) processes.

## Description

### BACKGROUND OF THE INVENTION

The present invention generally relates to Group 2 metal-organic complexes and their liquid compositions which are suitable for use to deposit a layer of the Group 2 metal-containing oxide thereof in chemical vapor deposition processes such as, for example, cyclic chemical vapor deposition (CCVD) or atomic layer deposition (ALD) on a semi-fabricated semiconductor substrate. More particularly, the Group 2 metal-organic complexes comprise polydentate β-ketoiminate ligands that are organic in character and are stable at ambient conditions.

In the semiconductor industry there is a growing need for volatile sources of different metal precursors to be used in the chemical vapor deposition (CVD) of Group 2 alkaline earth metal-containing oxide films and the like. The key property required for such metal sources is that they readily evaporate or sublime to give a metal containing vapor which can be decomposed in a controlled manner to deposit a film onto a target substrate. In this application, as well as for other applications, it is desirable that the alkaline earth compounds have a high volatility, which facilitates sublimation and the transport to the place of deposition, that they can deposit films thermally with or without oxidizing agents at relatively low temperatures, such as those of the order of 450°C, and that, for certain application areas, they are deposited in the form of the oxides and not, for example, in the form of other decomposition products such as carbonates.

Alkaline earth metal β-diketoiminates and certain derivatives have already been used for the CVD method. Complexes of alkaline earth metals and multidentate ligands have been employed in the prior art as precursors, however, such complexes are often present as oligomers whose volatility and stability are often unsatisfactory. Accordingly, the semiconductor fabrication industry continues to demand novel metal sources containing precursors for CVD processes including atomic layer deposition ("ALD") for fabricating conformal metal containing films on substrates such as silicon, metal nitride, metal oxide and other metal-containing layers using these metal-containing precursors.

### BRIEF SUMMARY OF THE INVENTION

In a first aspect, the present invention provides a metal-containing polydentate β-ketoiminate selected from: 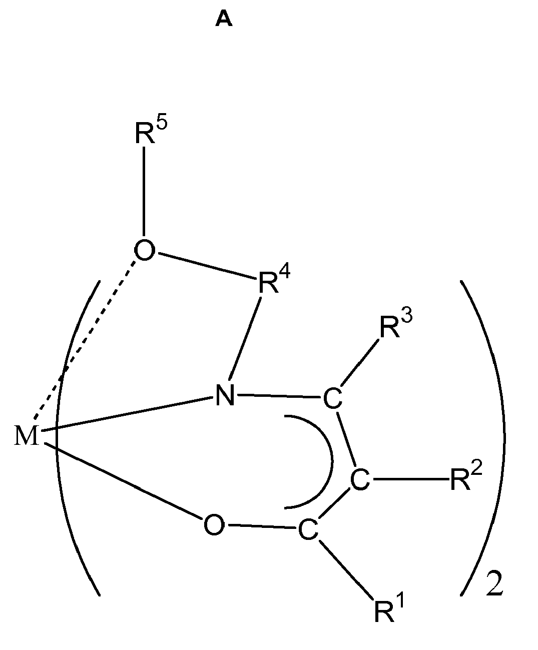 wherein M is a Group 2 metal selected from: magnesium, calcium, strontium, and barium; R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl; R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl; R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl; R⁴ is a C₁ to C₆ linear or branched alkylene; and R⁵ is selected from: a C₁ to C₁₀ alkyl, a C₁ to C₁₀ fluoroalkyl, a C₃ to C₁₀ cycloaliphatic, and a C₆ to C₁₀ aryl, 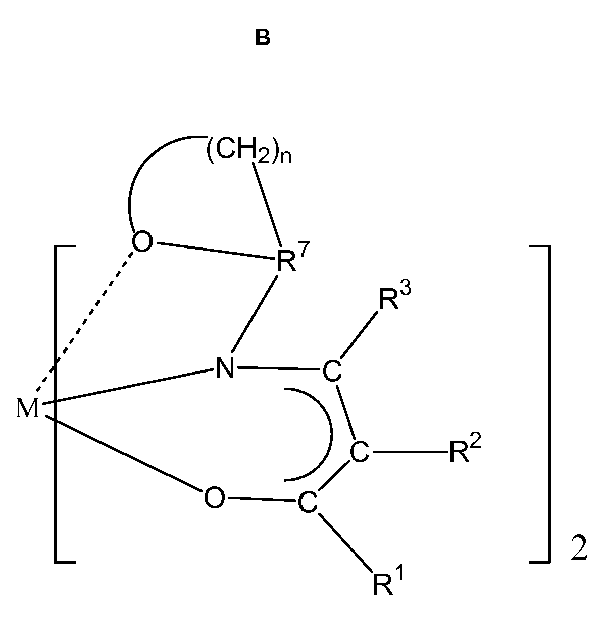 wherein M is a Group 2 metal selected from: magnesium, calcium, strontium, and barium; R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl; R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl; R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl; R⁷ is an organic group comprising 2 or 3 carbon atoms, thus making a five- or six-member coordinating ring to the metal center; and n = 3, 4, 5, and 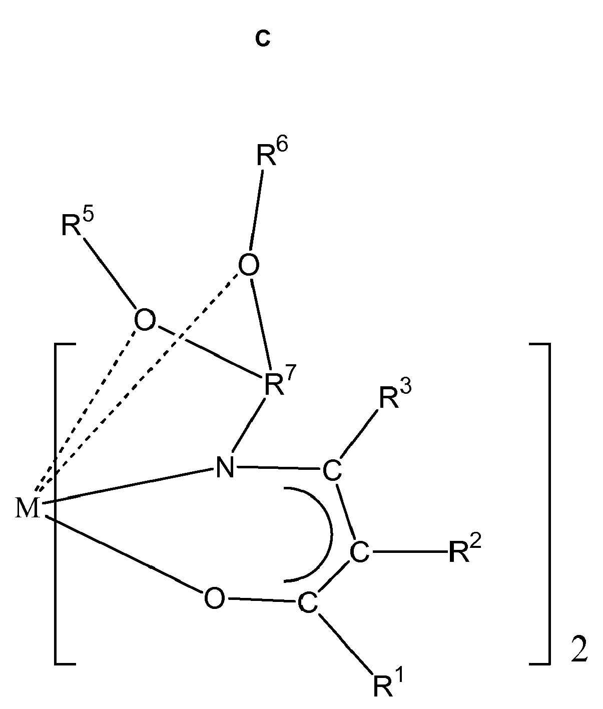 wherein M is a Group 2 metal selected from: magnesium, calcium, strontium, and barium; R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl; R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl; R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl; R⁷ an organic group comprising 2 or 3 carbon atoms; and R⁵ and R⁶ are each independently selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl.

The metal-containing polydentate β-ketoiminate of Structure A, B or C may be in a monomeric, dimeric, oligomeric or polymeric form. Preferably, the metal-containing polydentate β-ketoiminate is in a monomeric form (as depicted above).

In a preferred embodiment, for each of Structures A, B, and C, R¹ is a C₄ to C₆ alkyl group. More preferably, R¹ is selected from: tert-butyl, iso-butyl, sec-butyl, and tert-pentyl.

In a preferred embodiment, for each of Structures A, B, and C, M is selected from strontium and barium. More preferably, for each of Structures A, B, and C, M is barium.

In a preferred embodiment, for Structure A, R⁴ is either a linear alkylene bridge containing 2 or 3 carbon atoms or a branched alkylene bridge containing 3 or 4 carbon atoms and having at least one chiral carbon atom.

In a preferred embodiment, for each of Structures B and C, R⁷ is either

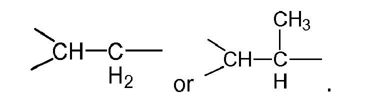

In a preferred embodiment, the metal-containing polydentate β-ketoiminate is selected from: bis(2,2-dimethyl-5-[(2-methoxyethyl)amino]hex-4-en-3-onato)strontium, bis(2,2-dimethyl-5-[(2-methoxy-1-methylethyl)amino]hex-4-en-3-onato)strontium, bis(,2-dimethyl-5-[(2-methoxy-1-methylethyl)amino]hex-4-en-3-onato)strontium, bis(2,2-dimethyl-5-[(2,2-dimethoxyethyl)amino]hex-4-en-3-onato)strontium, bis(2,2-dimethyl-5-[(2,2-dimethoxyethyl)amino]hex-4-en-3-onato)barium, and bis(2,2-dimethyl-5-[(tetrahydrofuran-2-ylmethyl)amino]hex-4-en-3-onato)strontium.

In a second aspect, the present invention provides a metal-containing polydentate β-ketoiminate as defined above that is free of fluoroalkyl groups. Preferred embodiments of the second aspect are as defined above in relation to the first aspect.

In a third aspect, the present invention provides a metal-containing polydentate β-ketoiminate that is the reaction product of a polydentate β-ketoiminate ligand and a metal alkoxide or metal amide, wherein the metal is selected from magnesium, calcium, strontium and barium, and the polydentate β-ketoiminate ligand is selected from: 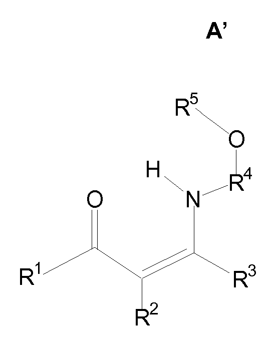 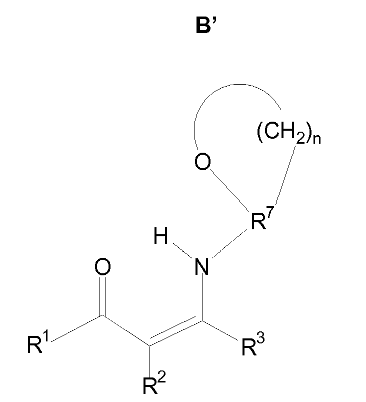 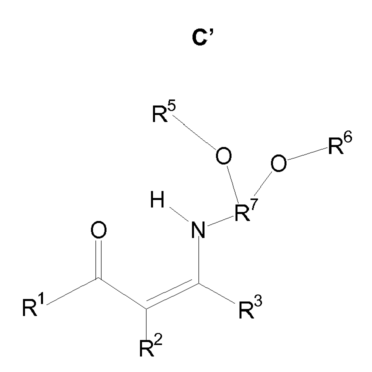 wherein: in Structure A', R¹ to R⁵ are the same as R¹ to R⁵, respectively, of Structure A according to the first aspect; in Structure B', R¹ to R³ and R⁷ are the same as R¹ to R³ and R⁷, respectively, of Structure B according to the first aspect; and in Structure C', R¹ to R³, R⁵ and R⁶ are the same as R¹ to R³, R⁵ and R⁶, respectively, of Structure C according to the first aspect. In preferred embodiments of the third aspect, R¹ to R⁷ may be as further defined in relation to the second aspect or preferred embodiments of the first or second aspect as described above (and/or subsequently).

In a fourth aspect, the present invention provides a liquid composition suitable for use to deposit a Group 2 metal-containing film on a substrate by a vapor deposition process, the composition comprising: a metal-containing polydentate β-ketoiminate according to the first, second or third aspect; and a solvent selected from: aliphatic hydrocarbons, aromatic hydrocarbons, ethers, esters, nitriles, organic esters, organic amines, polyamines, organic amides, alcohols, and mixtures thereof.

In a preferred embodiment, the solvent is a solvent selected from glyme solvents having from 1 to 20 ethoxy -(C₂H₄O)- repeat units; C₂-C₁₂ alkanols, organic ethers selected from dialkyl ethers comprising C₁-C₆ alkyl moieties, C₄-C₈ cyclic ethers; C₁₂-C₆₀ crown O₄-O₂₀ ethers wherein the prefixed Cᵢ range is the number i of carbon atoms in the ether compound and the suffixed Oᵢ range is the number i of oxygen atoms in the ether compound; C₆-C₁₂ aliphatic hydrocarbons; C₆-C₁₈ aromatic hydrocarbons; and organic amides of the form RCONR'R" wherein R and R' are alkyl having from 1-10 carbon atoms and they can be connected to form a cyclic group (CH₂)ₙ, wherein n is from 4-6, preferably 5, and R" is selected from alkyl having from 1 to 4 carbon atoms and cycloalkyl. Preferably, the solvent is an organic amide selected from: N-methyl- or N-ethyl- or N-cyclohexyl-2-pyrrolidinones, *N,N*-diethylacetamide, and *N,N-*diethylformamide.

In a fifth aspect, the present invention provides a vapor deposition process for forming a Group 2 metal oxide layer on a substrate, the process comprising the steps of: dissolving a metal-containing polydentate β-ketoiminate according to the first, second or third aspect in a solvent, wherein the solvent is selected from: aliphatic hydrocarbons, aromatic hydrocarbons, ethers, esters, nitriles, organic esters, organic amines, polyamines, organic amides, alcohols, and mixtures thereof; and depositing a layer of the Group 2 metal-containing film on a substrate.

In a preferred embodiment, the vapor deposition process is selected from chemical vapor deposition and atomic layer deposition.

In a preferred embodiment, the process employs an oxygen containing agent, preferably an oxygen containing agent selected from water, O₂, H₂O₂, ozone, water plasma, oxygen plasma, and mixtures thereof.

In a sixth aspect, the present invention provides a vapor deposition process for forming a conformal metal oxide thin film on a substrate wherein a precursor source and an oxygen containing agent are introduced to a deposition chamber and a metal oxide film deposited on a substrate, which process comprises using the metal containing complex of the first, second or third aspect as the precursor source.

In a seventh aspect, the present invention provides a method for making a metal-containing polydentate β-ketoiminate comprising reacting a polydentate β-ketoiminate ligand with a metal alkoxide in an organic solvent or a mixture of solvents.

In a preferred embodiment, the metal is selected from magnesium, calcium, strontium and barium.

In a preferred embodiment, the polydentate β-ketoiminate ligand is selected from Structures A', B' and C' as defined above in relation to the third aspect.

In a preferred embodiment, the organic solvent is selected from: THF, toluene, hexanes, ether, and mixtures thereof.

In a preferred embodiment (according to the third and/or seventh aspect), the metal alkoxide comprises an alkoxide selected form: methoxide, ethoxide, iso-propoxide, n-propoxide, tert-butoxide, sec-butoxide, iso-butoxide, and mixtures thereof. Preferably, the metal alkoxide is a metal iso-propoxide (such as strontium iso-propoxide or barium iso-propoxide).

In a preferred embodiment (according to the third and/or seventh aspect), the polydentate β-ketoiminate is selected from: 2,2-dimethyl-5-[(2-methoxyethyl)amino]hex-4-en-3-one, 2,2-dimethyl-5-[(2-methoxy-1-methylethyl)amino]hex-4-en-3-one, 4-[(2,2-dimethoxyethyl)amino]pent-3-en-2-one, 2,2-dimethyl-5-[(2,2-dimethoxyethyl)amino]hex-4-en-3-one, and 2,2-dimethyl-5-[(tetrahydrofuran-2-ylmethyl)amino]hex-4-en-3-one.

### BRIEF DESCRIPTION OF SEVERAL VIEWS OF THE DRAWINGS

Figure 1 is a crystal structure of bis(2,2-dimethyl-5-[(2-methoxyethyl)amino]hex-4-en-3-onato)strontium;
Figure 2 is a TGA of bis(2,2-dimethyl-5-[(2-methoxyethyl)amino]hex-4-en-3-onato)strontium (solid line) vs. 2,2-dimethyl-5-[(2-methoxyethyl)amino]hex-4-en-3-one (dashed line);
Figure 3 is a crystal structure of bis(2,2-dimethyl-5-[(2-methoxy-1-methylethyl)amino]hex-4-en-3-onato)strontium;
Figure 4 is a TGA of bis(2,2-dimethyl-5-[(2-methoxy-1-methylethyl)amino]hex-4-en-3-onato)strontium; and
Figure 5 is a crystal structure of bis(2,2-dimethyl-5-[(2,2-dimethoxyethyl)amino]hex-4-en-3-onato)barium.

### DETAILED DESCRIPTION OF THE INVENTION

This invention is related to Group 2 metal-containing polydentate β-ketoiminate precursors and compositions comprising Group 2 metal-containing polydentate β-ketoiminate precursors, wherein the polydentate β-ketoiminate precursors incorporate an ether or alkoxy group in the imino portion of the molecule. The compounds and compositions are useful for fabricating metal containing films on substrates such as silicon, metal nitride, metal oxide and other metal layers via chemical vapor deposition (CVD) processes. As used herein, the term "chemical vapor deposition processes" refers to any process wherein a substrate is exposed to one or more volatile precursors, which react and/or decompose on the substrate surface to produce the desired deposition. Examples include plasma enhanced chemical vapor deposition (PECVD), low pressure chemical vapor deposition (LPCVD), direct liquid injection chemical vapor deposition (DLCVD), hot wire chemical vapor deposition (HWCVD), cyclic chemical vapor deposition (CCVD), molecular layer deposition (MLD), atomic layer deposition (ALD), and metal-organic chemical vapor deposition (MOCVD). The deposited metal films (which includes Group 2 metal oxide films) have applications ranging from computer chips, optical device, magnetic information storage, to metallic catalyst coated on a supporting material.

The metal-containing multidentate β-ketoiminate precursors of the present invention comprise Group 2 metals of the Periodic Table of Elements. The Group 2 metals include beryllium (Be), magnesium (Mg), calcium (Ca), strontium (Sr), barium (Ba) and radium (Ra). In preferred embodiments of the present invention, the Group II metal is calcium, strontium, or barium. In more preferred embodiments of the present invention, the Group 2 metal is strontium or barium.

The multidentate polydentate β-ketoiminates according to the present invention preferably incorporate an alkoxy group in the imino group. The multidentate polydentate β-ketoiminates are selected from the group represented by the Structures A, B, and C. Structure A is defined as 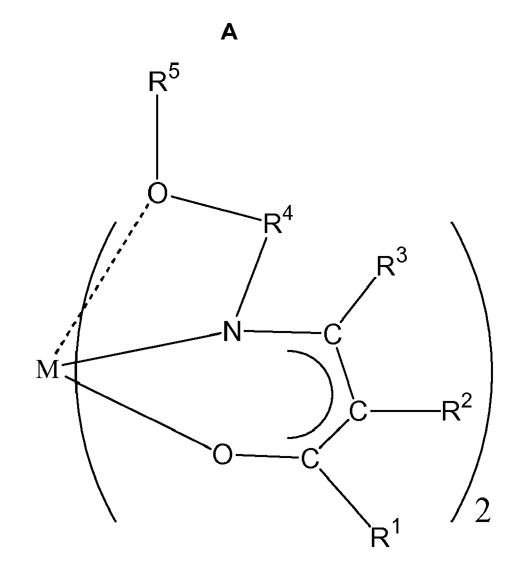 wherein M is a Group 2 metal such as, for example, magnesium, calcium, strontium, and barium. Preferably, M is strontium or barium. The organo groups (i.e., the R groups) employed in the complexes of the present invention may include a variety of organo groups and they may be linear or branched. In preferred embodiments, R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl. As used herein, the group "alkoxyalkyl" refers to an ether-like moiety that includes a C-O-C fragment. Examples include - CH₂CH₂-O-CH₂CH₂-O-CH₃ and -CH₂CH₂-O-CH₂-O-CH₃. Preferably, R¹ is a bulky (for example, branched) alkyl group containing 4 to 6 carbon atoms such as, for example, a *tert*-butyl group, an *iso*-butyl, a *sec*-butyl, and a *tert*-pentyl group. The most preferred R¹ group is *tert*-butyl or *tert*-pentyl. Preferably, R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl. More preferably, R² is hydrogen, or a C₁ to C₂ alkyl. Preferably, R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl. More preferably, R³ is a C₁ to C₂ alkyl. Preferably, R⁴ is a C₁ to C₆ linear or branched alkylene and, more preferably, R⁴ is a linear alkylene bridge containing 2 or 3 carbon atoms or is a branched alkylene bridge containing 3 or 4 carbon atoms and having at least one chiral center carbon atom. Without intending to be bound by a particular theory, it is believed that the chiral center in the ligand plays a role in lowering the melting point as well as increasing the thermal stability of the complex. Preferably, R⁵ is selected from: a C₁ to C₁₀ alkyl, a C₁ to C₁₀ fluoroalkyl, a C₃ to C₁₀ cycloaliphatic, and a C₆ to C₁₀ aryl. More preferably, R⁵ is a C₁ to C₂ alkyl.

Structure B is defined as 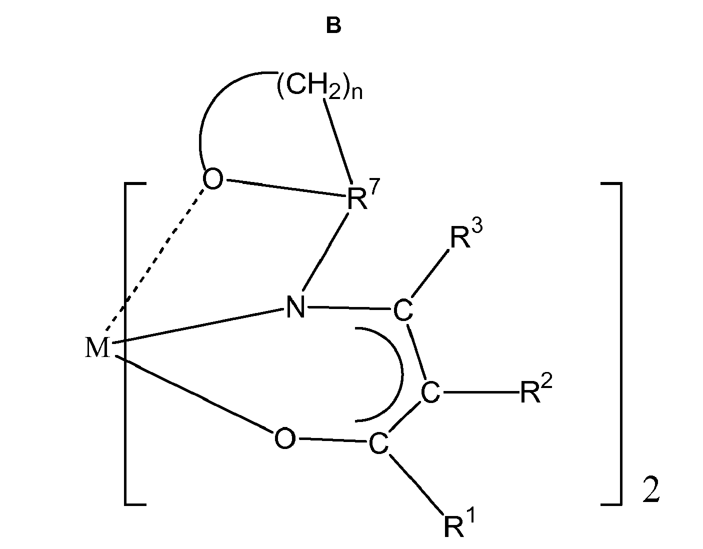 wherein M is a Group 2 metal selected from magnesium, calcium, strontium, barium, and n is 3, 4, 5, or 6. Preferably, M is strontium or barium. The organo groups (i.e., the R groups) employed in the complexes of the present invention may include a variety of organo groups and they may be linear or branched. In preferred embodiments, R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl. Preferably, R¹ is a bulky (for example, branched) alkyl group containing 4 to 6 carbon atoms such as, for example, a *tert*-butyl group, an iso-butyl group, a sec-butyl group, and a *tert*-pentyl group. The most preferred R¹ group is *tert*-butyl or *tert*-pentyl. Preferably, R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl. More preferably, R² is hydrogen, a C₁ to C₂ alkyl. Preferably, R³ is selected from: a C₁ to C₁₀ alkyl, a C₁ to C₁₀ alkoxyalkyl, a C₁ to C₁₀ alkoxy, a C₃ to C₁₀ cycloaliphatic and a C₆ to C₁₀ aryl. More preferably, R³ is a C₁ to C₂ alkyl. Preferably, R⁷ is a C₁ to C₆ linear or branched alkylene and, more preferably, R⁷ is an organic group comprising 2 or 3 carbon atoms, thus making a five- or six-member coordinating ring to the metal center. Preferably, n=3, 4, or 5. In preferred embodiments, R⁷ is

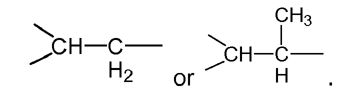

Structure C is defined as 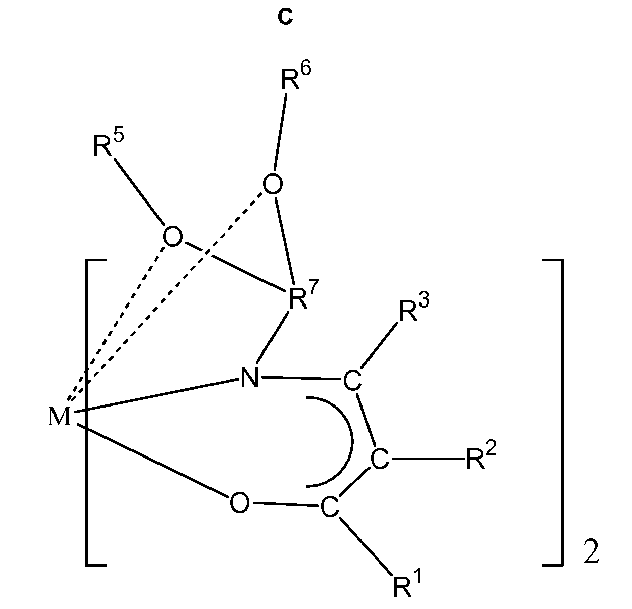 wherein M is a Group 2 metal selected from magnesium, calcium, strontium, and barium. Preferably, M is strontium or barium. The organo groups (i.e., the R groups) employed in the complexes of the present invention may include a variety of organo groups and they may be linear or branched. In preferred embodiments, R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl. Preferably, R¹ is a bulky (for example, branched) alkyl group containing 4 to 6 carbon atoms such as, for example, a *tert*-butyl group, iso-butyl group, sec-butyl group, and a *tert*-pentyl group. The most preferred R¹ group is *tert*-butyl or *tert*-pentyl. Preferably, R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl. More preferably, R² is hydrogen or a C₁ to C₂ alkyl. Preferably, R³ is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl. More preferably, R³ is a C₁ to C₂ alkyl. Preferably, R⁷ is selected from: a C₂ to C₆ linear or branched alkylene, a C₁ to C₁₀ fluoroalkylene, and a C₆ to C₁₀ aryl. R⁷ may, for example, be a branched alkylene bridge containing 3 or 4 carbon atoms and having at least one chiral carbon atom. More preferably, R⁷ is an organic group comprising 2 or 3 carbon atoms, thus making a five- or six-member coordinating ring to the metal center. In preferred embodiments, R⁷ is 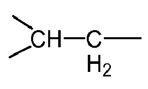 or 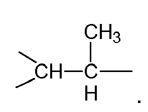 Preferably, R⁵ and R⁶ are selected from : a C₁ to C₁₀ alkyl, a C₁ to C₁₀ fluoroalkyl, a C₃ to C₁₀ cycloaliphatic, and a C₆ to C₁₀ aryl. More preferably, R⁵ and R⁶ are selected from C₁ to C₂ alkyl.

In preferred embodiments of the present invention, the compounds of Structures A, B, and C as defined above are free of fluoroalkyl groups.

The polydentate β-ketoiminate ligands according to the present invention can be prepared by well known procedures such as the Claisen condensation of a bulky ketone and an ethyl ester in presence of a strong base such as, for example, sodium amide or hydride to provide a diketone, followed by another known procedure such as Schiff base condensation reaction with alkoxyalkylamine. The Schiff base condensation reaction, for example, is shown below:

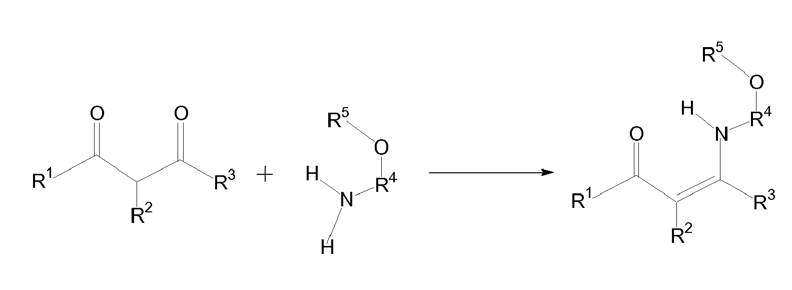

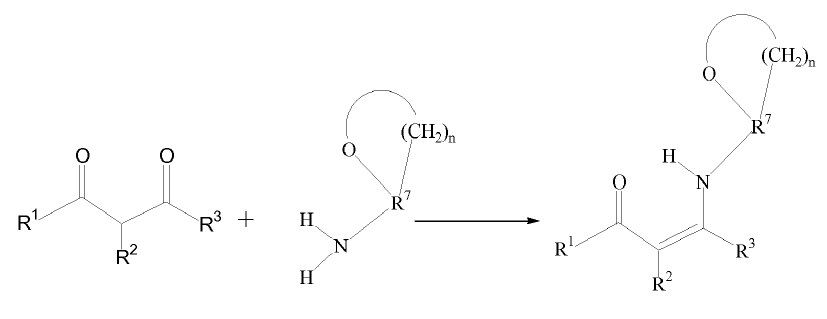

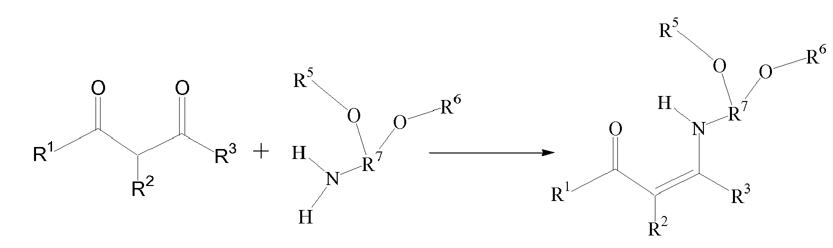

The resulting ligands can be purified via vacuum distillation for a liquid or crystallization for solid. As a discussed above, it is preferred to employ a bulky R¹ group, e.g., C₄₋₁₀ branched alkyl groups without hydrogen attached to the carbon connected to the ketone functionality because the bulky R¹ group prevents any side reactions occurring in the Schiff condensation and later protecting the metal centers from inter-molecular interaction. It should be noted, however, that the R¹⁻⁶ groups in the polydentate ligands should be as small as possible in order to decrease the molecular weight of the resulting metal-containing complexes which will allow the complexes to have a sufficiently high vapor pressure.

The metal-containing complexes of the present invention can then be prepared via the reaction of the multidentate ketoiminato ligands with metal alkoxide in a solvent as shown below:

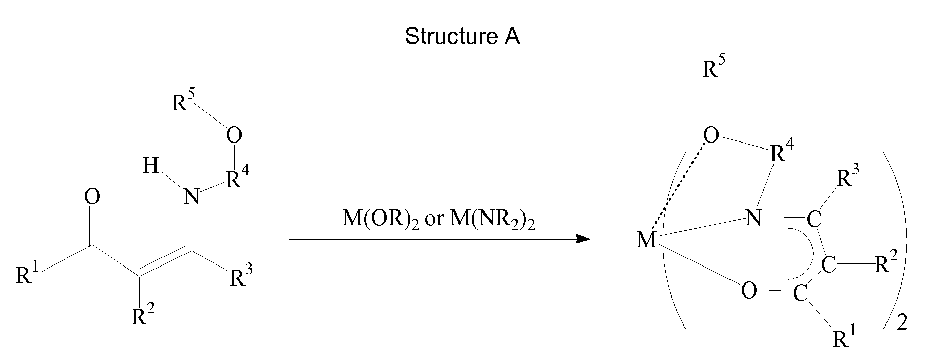

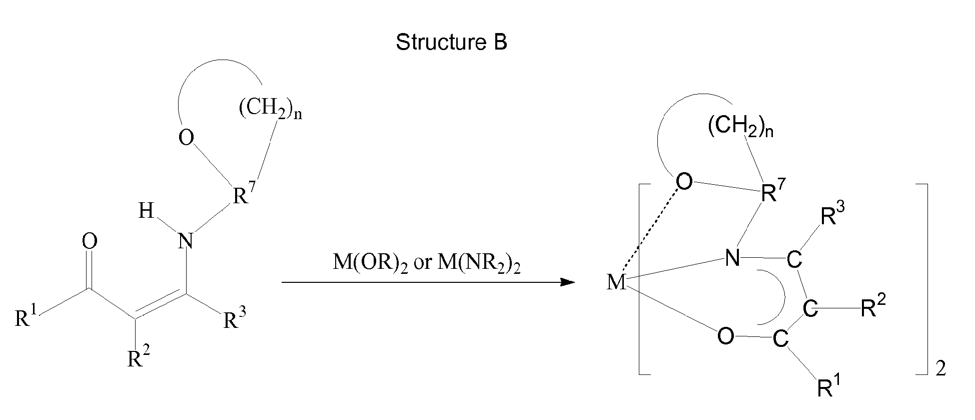

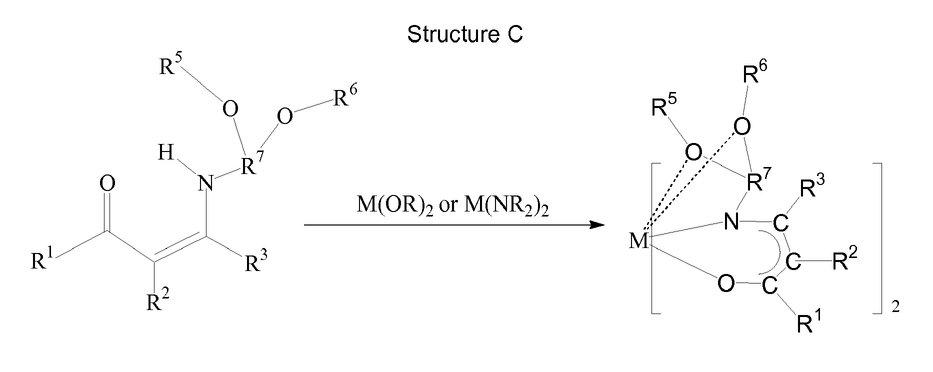

The metal, M, is as defined above. Preferably, the oxide portion of the metal alkoxide is selected form: methoxide, ethoxide, iso-propoxide, n-propoxide, tert-butoxide, sec-butoxide, iso-butoxide, and mixtures thereof. In preferred embodiments, the metal alkoxide is strontium iso-propoxide.

Suitable solvents for use in the reaction according to the present invention include, for example, THF, toluene, hexane, an ether, and mixtures thereof. THF is the preferred solvent. In preferred embodiments, the reaction according to the present invention occurs at a temperature of from 10°C to 150°C. In preferred embodiments, the reaction occurs under ambient temperature or reflux at the boiling point of the solvent.

The compounds of each of Structures A, B, and C are solids at room temperature which can be purified via crystallization or sublimation.

Group 2 metal-containing complexes with multidentate β-ketoiminate ligands according to the present invention may be employed as precursors to make thin metal oxide films via either the CVD, CCVD or ALD techniques at temperatures less than 500°C. The CVD process can be carried out with or without oxidizing agents whereas an ALD or CCVD process usually involves the employment of another reactant such as an oxidizing agent.

Oxidizing agents for chemical vapor deposition or atomic layer deposition processes include oxygen, hydrogen peroxide and ozone and plasma oxygen. For multicomponent metal oxides, the Group 2 metal-containing complexes according to the present invention can be premixed if they have the same multidentate β-ketoiminate ligands. These Group 2 metal-containing complexes with multidentate β-ketoiminate ligands can be delivered in vapor phase into, for example, a CVD or ALD reactor via well-known bubbling or vapor draw techniques. A direct liquid delivery method can also be employed by dissolving the complexes in a suitable solvent or a solvent mixture to prepare a solution with a molar concentration from 0.001 to 2 M depending the solvent or mixed-solvents employed.

The solvent employed in solubilizing the precursor for use in a deposition process may comprise any compatible solvent or their mixture including aliphatic hydrocarbons, aromatic hydrocarbons, ethers, esters, nitriles, and alcohols. The solvent component of the solution preferably comprises a solvent selected from glyme solvents having from 1 to 20 ethoxy -(C₂H₄O)- repeat units; C₂-C₁₂ alkanols, organic ethers selected from dialkyl ethers comprising C₁-C₆ alkyl moieties, C₄-C₈ cyclic ethers; C₁₂-C₆₀ crown O₄-O₂₀ ethers wherein the prefixed Cᵢ range is the number i of carbon atoms in the ether compound and the suffixed Oᵢ range is the number i of oxygen atoms in the ether compound; C₆-C₁₂ aliphatic hydrocarbons; C₆-C₁₈ aromatic hydrocarbons; organic esters; organic amines, polyamines and organic amides.

Another class of solvents that offers advantages is the organic amide class of the form RCONR'R" wherein R and R' are alkyl having from 1-10 carbon atoms and they can be connected to form a cyclic group (CH₂)ₙ, wherein n is from 4-6, preferably 5, and R" is selected from alkyl having from 1 to 4 carbon atoms and cycloalkyl. N-methyl- or N-ethyl- or N-cyclohexyl-2-pyrrolidinones, *N,N*-Diethylacetamide, and *N,N-*Diethylformamide are examples.

In one embodiment of the method of the present invention, a cyclic deposition process such as CCVD, ALD, or PEALD may be employed, wherein a Group 2 metal-containing complex or its solution and an oxidizer such as, for example, ozone, oxygen plasma or water plasma are employed. The gas lines connecting from the precursor canisters to the reaction chamber are heated to about 190°C, and the container of the Group 2 metal-containing complex is kept at about 190°C for bubbling whereas the solution is injected into a vaporizer kept at 180°C for direct liquid injection. 100 sccm of argon gas is preferably employed as a carrier gas to help deliver the vapor of the Group 2 metal-containing complex to the reaction chamber during the precursor pulsing. The reaction chamber process pressure is about 1Torr (130 Pa). In a typical ALD or CCVD process, the substrate such as silicon oxide or metal nitride are heated on a heater stage in a reaction chamber that is exposed to the Group 2 metal-containing complex initially to allow the complex to chemically adsorb onto the surface of the substrate. An inert gas such as argon gas purges away unadsorbed excess complex from the process chamber. After sufficient Ar purging, an oxygen source is introduced into reaction chamber to react with the absorbed surface followed by another inert gas purge to remove reaction by-products from the chamber. The process cycle can be repeated to achieve the desired film thickness.

The following example illustrates the preparation of the metal-containing complexes with tridentate β-ketoiminate ligands as well as their use as precursors in metal-containing film deposition processes.

### EXAMPLES

The following example illustrates the preparation of the metal-containing complexes with tridentate β-ketoiminate ligands as well as their use as precursors in metal-containing film deposition processes.

### Example 1

### Synthesis of 2,2-dimethyl-5-[(2-methoxyethyl)amino]hex-4-en-3-one

To a solution of 15.00g (105.49mmol) 2,2-dimethyl-3,5-hexanedione in 100mL THF loaded with 18.00g (126.58mmol) sodium sulfate was added 9.51g (126.58mmol) 2-methoxyethylamine. The reaction mixture was heated at 50°C for several days after which THF was evaporated from mixture under vacuum yielding a yellow oil. Vacuum transfer of the residual oil heating at 130°C under 150mTorr (20 Pa) vacuum yielded 17.31g of a light yellow solid. The yield was 82%. ¹H NMR (500 MHz, C₆D₆): δ= 11.45 (s, 1H), 5.21 (s, 1H), 2.93 (s, 3H), 2.90 (t, 2H), 2.78 (q, 2H), 1.49 (s, 3H), 1.31 (s, 9H).

### Example 2

### Synthesis of 2,2-dimethyl-5-[(2-methoxy-1-methylethyl)amino]hex-4-en-3-one

A reaction flask loaded with 12.6 g of 2,2-dimethylhexan-3,5-dione with excess 1-methoxy-2-propylamine and excess anhydrous sodium sulfate in diethyl ether was stirred until the dione was no longer observed by GC-MS. A clear solution was obtained by filtration and the filtrand was washed with diethyl ether. The solvent and excess amine was removed from the combined diethyl ether solution via a rotary evaporator. 13.2 g of product was obtained by vacuum distillation at 600 mtorr (80 Pa), 96 C. ¹H NMR (500 MHz, C₆D₆): δ= 11.54 (s, 1H), 5.20 (s, 1H), 3.31 (m, 1H), 2.93 (s, 3H), 2.84 (m, 2H), 1.60 (s, 3H), 1.30 (s, 9H), 0.87 (d, 3H)

### Example 3

### Synthesis of 4-[(2,2-dimethoxyethyl)amino]pent-3-en-2-one

21.0g of aminoacetaldehyde dimethylacetal (0.2 moles) were dissolved into 100 ml of tetrahydrofuran to which 20.0g of 2,4-pentanedione (0.2 moles) were added dropwise over 5 minutes. The resulting mixture was then stirred overnight after which time the solvent and water of condensation formed during the reaction was removed by vacuum distillation. The final product was then vacuum distilled as a clear liquid. Yield of MeC(O)CH₂C(NCH₂CH(OMe)₂)Me = 25.0g (72% of theoretical). ¹H NMR : (500 MHz, C₆D₆): δ = 1.47(s, 3H), δ = 2.00(d, 3H), δ = 2.98 (t, 2H), δ = 3.05 (s, 6H), δ = 4.00 (t, 1H), δ = 4.89 (s, 1H), δ = 11.2 (bs, 1H); GCMS parent ion at 211 mu.

### Example 4

### Synthesis of 2,2-dimethyl-5-[(2,2-dimethoxyethyl)amino]hex-4-en-3-one

To a solution of 6.18g (43.46mmol) 2,2-dimethyl-3,5-hexanedione in 150mL toluene was added 5.48g (52.15mmol) aminoacetalhedyde dimethylacetal followed by 12.0g (84.48mmol) of sodium sulfate. The reaction was heated at reflux for several days after which toluene was distilled off heating at 142°C under static conditions. The resulting residual oil was heated to 75°C under 100mTorr (13.3 Pa) vacuum for one hour to remove any residual toluene. Vacuum transfer of residual oil heating at 102°C under 100mTorr (13.3 Pa) vacuum yielded a lime green oil weighing 7.17g with a yield of 71%. ¹H NMR (500 MHz, C₆D₆): δ= 11.43 (s, 1H), 5.21 (s, 1H), 4.02 (t, 1H), 3.03 (s, 6H), 2.99 (t, 2H), 1.50 (s, 3H), 1.29 (s, 9H).

### Example 5

### Synthesis of bis(2,2-dimethyl-5-[(2-methoxyethyl)amino]hex-4-en-3-onato)strontium

To a suspension of 0.94g (4.56mmol) Sr-isopropoxide in THF was added 2.00g (10.04mmol) 2,2-dimethyl-5-[(2-methoxyethyl)amino]hex-4-en-3-one in THF at room temperature. The reaction mixture turned to a solution in a couple of minutes which was stirred for 16 hours at room temperature. All volatiles were evaporated under vacuum to obtain a sticky yellow oil. After rinsing with cold (e.g., about -10°C) hexane, 2.57g of a sticky off-white solid was collected.

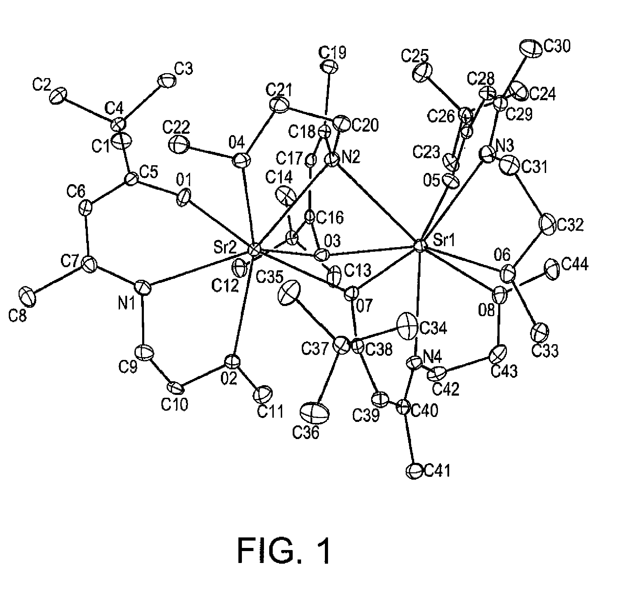

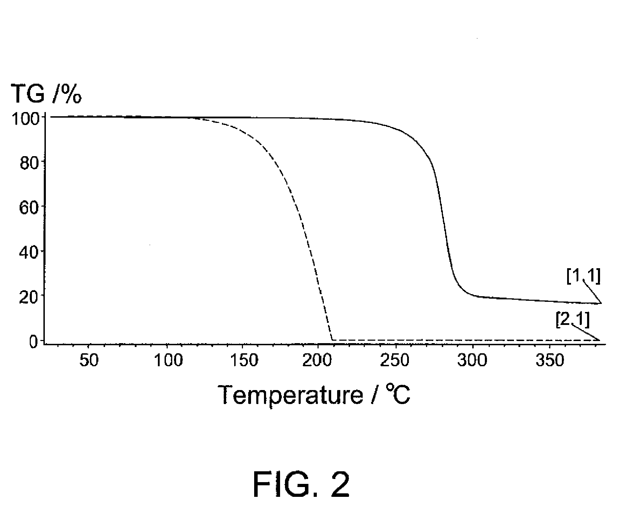

A single crystal of bis(2,2-dimethyl-5-[(2-methoxyethyl)amino]hex-4-en-3-onato)strontium was characterized by X-ray single crystal analysis, exhibiting that it is dimer in which one strontium atom is seven-coordinated with two nitrogen atoms and five oxygen atoms where the other eight-coordinated with three nitrogen atoms and five oxygen atoms. The structure of this compound is shown in Figure 1. Referring to Figure 2, the TGA of the complex indicates that it starts to vaporize at temperature greater than 250°C. There is about 15% residue, suggesting it is not a suitable precursor because it is not thermally stable. On the other hand, the pure ligand starts to vaporize at temperature greater than 120°C and completely vaporize at temperature of 200°C.

### Example 6

### Synthesis of bis(2,2-dimethyl-5-[(2-methoxy-1-methylethyl)amino]hex-4-en-3-onato)strontium

To a suspension of 1.00g (4.86mmol) Sr-isopropoxide in 20mL THF was added 1.95g (9.72mmol) 2,2-dimethyl-5-[(2-methoxy-1-methylethyl)amino]hex-4-en-3-one in 5mL THF. The reaction mixture was left to stir at room temperature for 16 hours in which the suspension became a homogenous solution. THF was evaporated under vacuum yielding a waxy white solid. Removal of all volatile under vacuum transfer yielded about 1.70g of off white solid. Recrystallization in octane gave colorless crystals with a yield of 72%. ¹H NMR (500 MHz, C₆D₆): δ= 5.10 (s, 1H), 3.34 (d, 1H), 3.05 (s, 3H), 2.75 (d, 1H), 1.79 (s, 3H), 1.42 (s, 9H), 1.02 (d, 3H).

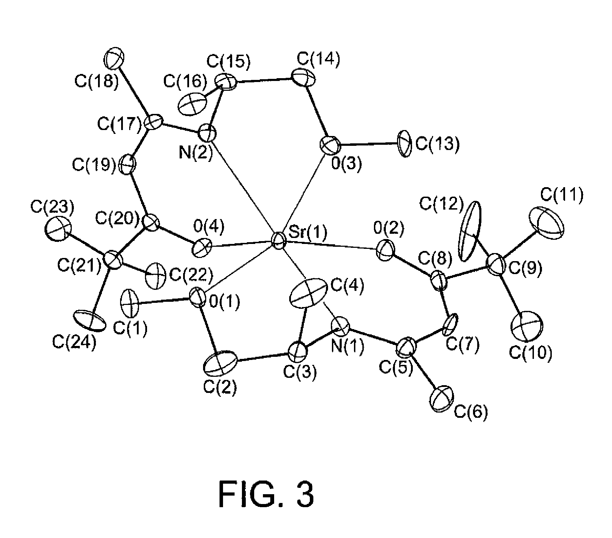

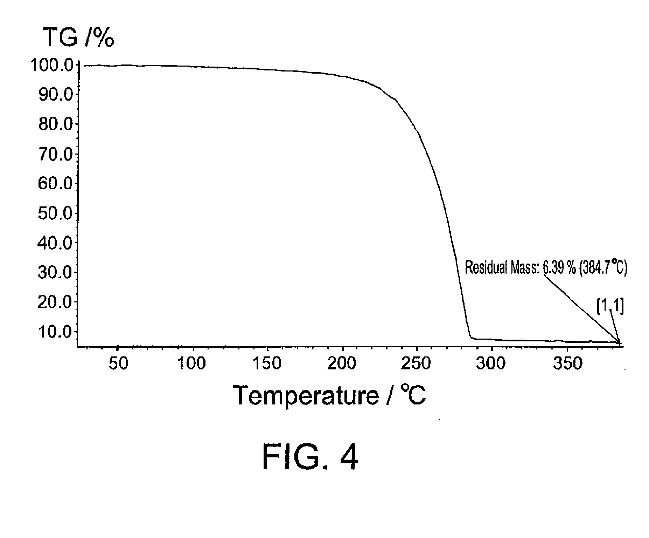

A single crystal of bis(2,2-dimethyl-5-[(2-methoxy-1-methylethyl)amino]hex-4-en-3-onato)strontium was characterized by X-ray single crystal analysis, exhibiting that it is a monomer in which the strontium atom is six-coordinated with two 2,2-dimethyl-5-[(2-methoxy-1-methylethyl)amino]hex-4-en-3-onato ligands. The structure of this compound is shown in Figure 3. Referring to Figure 4, the TGA analysis indicates that it is volatile with less than 5% residue, implying it can be employed as precursors in a CVD or ALD process.

### Example 7

### Synthesis of bis(4-[(2,2-dimethoxyethyl)amino]pent-3-en-2-onato)strontium

To a suspension of 0.50g (2.43mmol) Sr-isopropoxide in 15mL THF was added 0.90g (4.86mmol) 4-[(2,2-dimethoxyethyl)amino]pent-3-en-2-one in 5mL THF. The resulting suspension turned to a solution in approximately 10 minutes. The solution was stirred for 16 hours after which THF was evaporated under vacuum to give rise to about 1.20g of an amber oil. The proton NMR suggested there are some unreacted free ligand which makes the product oily. H NMR of solid crashed out from a hexanes solution of the oil indicated it is an oligomer.

### Example 8

### Synthesis of bis(2,2-dimethyl-5-[(2,2-dimethoxyethyl)amino]hex-4-en-3-onato)strontium

To a suspension of 1.00g (4.86mmol) Sr-isopropoxide in 25mL THF was added 2.33g (10.21 mmol) 2,2-dimethyl-5-[(2,2-dimethoxyethyl)amino]hex-4-en-3-one in 5mL THF. The resulting suspension turned to a solution in approximately 2 minutes which was left to stir at room temperature for 16 hours after which THF was evaporated from reaction mixture under vacuum to afford about 0.95g. ¹H NMR (500 MHz, C₆D₆): δ= 5.20 (s, 1H), 3.93 (t, 1H), 3.22 (d, 2H), 3.09 (s, 6H), 1.78 (s, 3H), 1.41 (s, 9H).

A single crystal of bis(2,2-dimethyl-5-[(2,2-dimethoxyethyl)amino]hex-4-en-3-onato)strontium was characterized by X-ray single crystal analysis, exhibiting that it is a one-dimensional polymer in which one strontium atom is eight-coordinated with two nitrogen atoms and six oxygen atoms.

### Example 9

### Synthesis of bis(2,2-dimethyl-5-[(2,2-dimethoxyethyl)amino]hex-4-en-3-onato)barium

To a clear solution of 0.96g (2.09mmol) Ba(N(SiMe3)2)2 in 20mL THF was added 0.96g (4.19mmol) 2,2-dimethyl-5-[(2,2-dimethoxyethyl)amino]hex-4-en-3-one in 5mL THF drop wise. Clear solution turned yellow and generated some evidence of heat and was stirred at room temperature for 3 hours. Removal of THF under vacuum provided a beige foam. 1.18g of off-white solid was collected via washing with anhydrous hexanes. ¹H NMR (500 MHz, C₆D₆): δ= 5.14 (s, 1H), 4.44 (b, 1H), 3.63 (b, 2H), 3.20 (s, 6H), 1.86 (s, 3H), 1.41 (s, 9H).

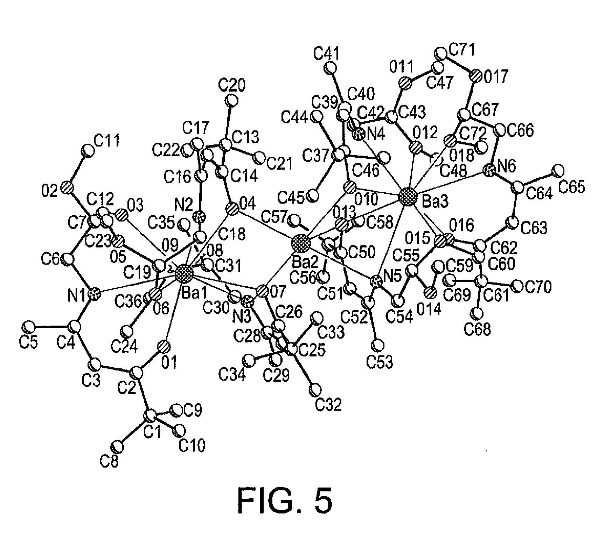

A single crystal of bis(2,2-dimethyl-5-[(2,2-dimethoxyethyl)amino]hex-4-en-3-onato)barium was characterized by X-ray single crystal analysis, exhibiting that it is trimer polymer in which one barium atom is six-coordinated with two nitrogen atoms and four oxygen atoms whereas the other two barium atoms are nine-coordinated with three nitrogen atoms and six oxygen atoms. The structure of this compound is shown in Figure 5.

### Example 10

### Synthesis of 2,2-dimethyl-5-[(tetrahydrofuran-2-ylmethyl)amino]hex-4-en-3-one

To a solution of 5.00g (35.16mmol) 2,2-dimethyl-3,5-hexanedione in 100mL THF was added 6.00g (42.24mmol) sodium sulfate followed by 4.27g (42.19mmol) tetrahydrofurfurylmethylamine. The reaction was refluxed for 16 hours after which GC indicated reaction was complete. All volatiles were evaporated off under vacuum for several hours. 4.0 g of yellow oil was collected via short-path distillation. The yield was 50%.

### Example 11

### Synthesis of bis (2,2-dimethyl-5-[(tetrahydrofuran-2-ylmethyl)amino]hex-4-en-3-oneato)strontium

To a suspension of 1.00g (4.86mmol) Sr-isopropoxide in 20mL THF was dropwise added 2.19g (9.72mmol) 2,2-dimethyl-5-[(tetrahydrofuran-2-ylmethyl)amino]hex-4-en-3-one in 5mL THF to give rise to a suspension which turned into a solution in approximately 2 minutes. The reaction was stirred for 16 hours after which yellow solution was evaporated under vacuum to afford a foam weighing 2.83g. ¹H NMR shows 50% conversion to product. ¹H NMR (500 MHz, C₆D₆): δ= 5.13 (s, 1H), 3.88 (b, 2H), 3.65 (m, 1H), 3.34 (b, 1H), 3.09 (b, 1H) 1.83 (s, 3H), 1.50 (m, 2H), 1.43 (s, 9H), 1.41 (m, 1H), 1.32 (m, 1H).

### Example 12

A silicon wafer is maintained at a temperature of 250°C in a reaction chamber at a pressure of 1.5 Torr (200 Pa). A Sr-containing compound of bis(2,2-dimethyl-5-[(2-methoxy-1-methylethyl)amino]hex-4-en-3-onato)strontium at 180°C is introduced into the chamber for 5 seconds with 100 sccm N₂. A purge of 200 sccm N₂ follows for 10 seconds. A dose of ozone is then introduced for 10 seconds. This is followed by a 10 second purge with 200 sccm of N₂. This procedure is repeated for 200 cycles to produces a film of 6 nm thickness. EDX confirmed the resulting film contains strontium and oxygen.

### Comparative Example 1

In this experiment, the procedure outlined in Example 7 of JP06298714 was followed. This example should be compared to Example 5 above.

3.34g (16.74mmol) of 2,2-dimethyl-5-[(2-methoxyethyl)amino]hex-4-en-3-one in 10mL THF was added dropwise to a suspension of 0.50g (5.58mmol) SrH₂ in 15mL THF. A minor amount of bubbling was evident. The reaction mixture was stirred at room temperature for 16 hours, which gave rise to a grey slurry. THF was evaporated under vacuum to yield a waxy off-white solid. The TGA of the solid indicated that it was the unreacted 2,2-dimethyl-5-[(2-methoxyethyl)amino]hex-4-en-3-one. The crude waxy solid was then subjected to vacuum transfer similar to what is described in the JP06898714 reference. A white volatile solid was collected, which was 2,2-dimethyl-5-[(2-methoxyethyl)amino]hex-4-en-3-one as its TGA matched that of pure 2,2-dimethyl-5-[(2-methoxyethyl)amino]hex-4-en-3-one. The TGA diagram in JP06898712 is consistent with that of pure 2,2-dimethyl-5-[(2-methoxyethyl)amino]hex-4-en-3-one. Accordingly, It is evident that what is disclosed in prior art JP06298714 for the preparation of bis(2,2-dimethyl-5-[(2-methoxyethyl)amino]hex-4-en-3-onato)strontium was unsuccessful. Instead, the ligand itself was actually isolated.

### Comparative Example 2

In this experiment, the procedure outlined in paragraph 52 of JP06298714 was followed.

3.34g (16.74mmol) of 2,2-dimethyl-5-[(2-methoxyethyl)amino]hex-4-en-3-one in 5mL THF was added dropwise to a suspension of 0.58g (4.23mmol) barium hydride in 20mL THF. The resulting slurry was stirred for 16 hours after which THF was evaporated under vacuum yielding 3.10g of a grainy liquid. Vacuum transfer at 150°C under 125mTorr (16.7 Pa) vacuum provided approximately 2.00g of a white solid that was characterized as 2,2-dimethyl-5-[(2-methoxyethyl)amino]hex-4-en-3-one, suggesting that the reaction did not occur.

**Table 1 Summary of Exemplary Complexes**

| | | | |
|---|---|---|---|
| Example 5 | R¹=Bu^{t}, R²=H, R³=Me, R⁴=-CH₂CH₂, R⁵=Me | M=Sr | Structure A |
| Example 6 | R¹=Bu^{t}, R²=H, R³=Me, R⁴=-CH(Me)CH₂, R⁵=Me | M=Sr | Structure A |
| Example 7 | R¹=Me, R²=H, R³=Me, R⁷=-CH₂CH, R⁵=R⁶=Me | M=Sr | Structure C |
| Example 8 | R¹=Bu^{t}, R²=H, R³=Me, R⁷=-CH₂CH, R⁵=R⁶=Me | M=Sr | Structure C |
| Example 9 | R¹=Bu^{t}, R²=H, R³=Me, R⁷=-CH₂CH, R⁵=R⁶=Me | M=Ba | Structure C |
| Example 11 | R¹=Bu^{t}, R²=H, R³=Me, R⁷=-CH₂CH, n=3 | M=Sr | Structure B |

The foregoing examples as summarized in Table 1 and description of the preferred embodiments should be taken as illustrating, rather than as limiting the present invention as defined by the claims.

## Claims

1. A polydentate β-ketoiminate selected from: 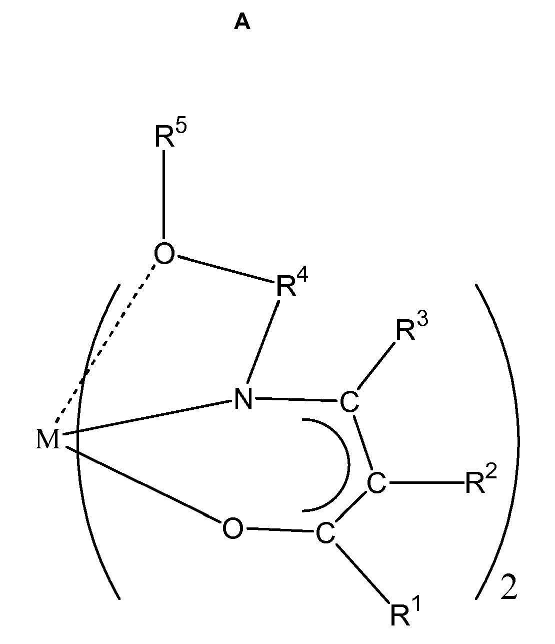 wherein:
M is a group 2 metal selected from: magnesium, calcium, strontium, and barium;
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁴ is a C₁ to C₆ linear or branched alkylene; and
R⁵ is selected from: a C₁ to C₁₀ alkyl, a C₁ to C₁₀ fluoroalkyl, a C₃ to C₁₀ cycloaliphatic, and a C₆ to C₁₀ aryl; 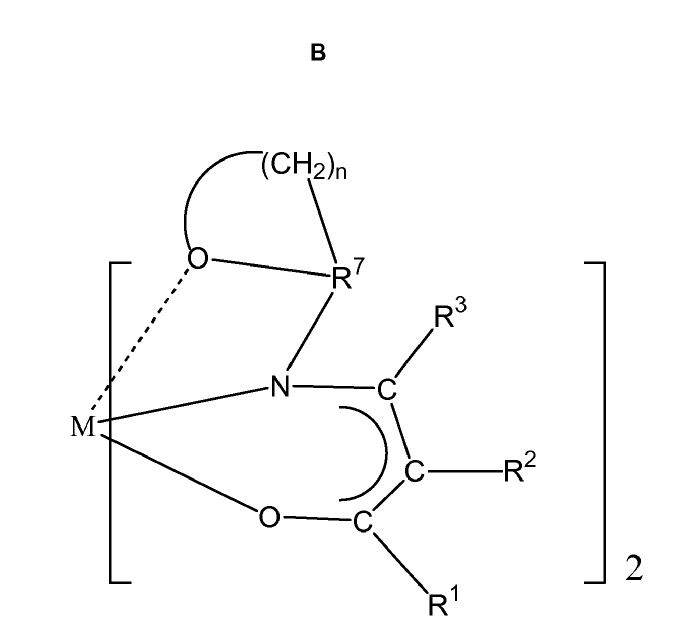
wherein:
M is a group 2 metal selected from: magnesium, calcium, strontium, and barium;
R' is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁷ is an organic group comprising 2 or 3 carbon atoms; and
n = 3, 4, or 5; and 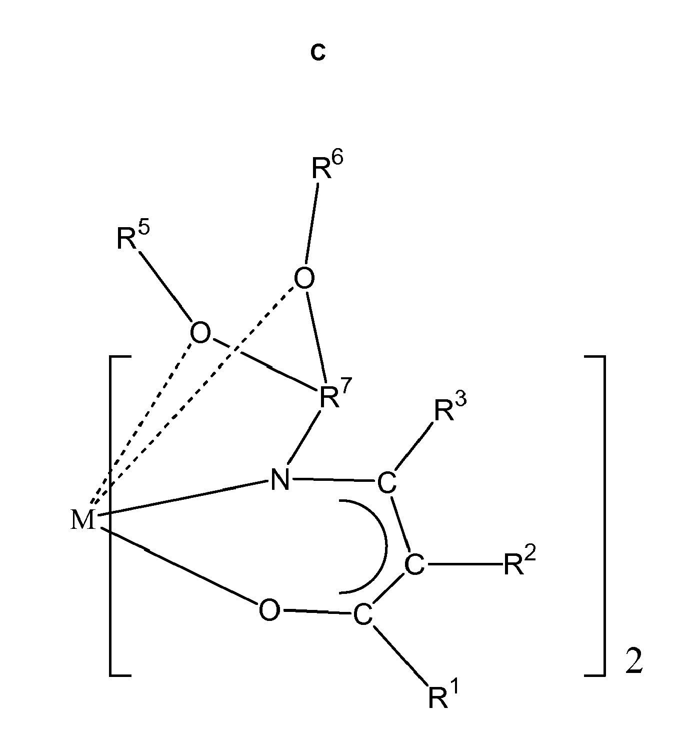
wherein:
M is a group 2 metal selected from: magnesium, calcium, strontium, and barium;
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R' is an organic group comprising 2 or 3 carbon atoms; and
R⁵ and R⁶ are each independently selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl; and
wherein each of Structures A, B and C may be in a monomeric, dimeric, oligomeric or polymeric form.

2. The polydentate β-ketoiminate of Claim 1,
wherein where the polydentate β-ketoiminate is of Structure A:
M is a group 2 metal selected from: magnesium, calcium, strontium, and barium;
R¹ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁴ is a C₁ to C₆ linear or branched alkylene; and
R⁵ is selected from: a C₁ to C₁₀ alkyl, a C₃ to C₁₀ cycloaliphatic, and a C₆ to C₁₀ aryl;
wherein where the polydentate β-ketoiminate is of Structure B:
M is a group 2 metal selected from: magnesium, calcium, strontium, and barium;
R¹ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁷ is an organic group comprising 2 or 3 carbon atoms; and
n = 3, 4, or 5; and
wherein where the polydentate β-ketoiminate is of Structure C:
M is a group 2 metal selected from: magnesium, calcium, strontium, and barium;
R¹ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁷ is an organic group comprising 2 or 3 carbon atoms; and
R⁵ and R⁶ are each independently selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl.

3. The polydentate β-ketoiminate of Claim 1 or 2 wherein, for each of Structures A, B, and C, R¹ is a C₄ to C₆ alkyl group.

4. The polydentate β-ketoiminate of Claim 3 wherein the C₄ to C₆ alkyl group, R¹ is selected from: tert-butyl, iso-butyl, sec-butyl, and tert-pentyl.

5. The polydentate β-ketoiminate of any preceding claim wherein, for each of Structures A, B, and C, M is selected from: strontium and barium.

6. The polydentate β-ketoiminate of any preceding claim wherein, for Structure A, R⁴ is either a linear alkylene bridge containing 2 or 3 carbon atoms or a branched alkylene bridge containing 3 or 4 carbon atoms and having at least one chiral carbon atom.

7. The polydentate β-ketoiminate of any one of Claims 1 to 5 wherein, for each of Structures B and C, R⁷ is either

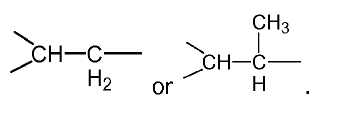

8. The polydentate β-ketoiminate of Claim 1 selected from: bis(2,2-dimethyl-5-[(2-methoxyethyl)amino]hex-4-en-3-onato)strontium, bis(2,2-dimethyl-5-[(2-methoxy-1-methylethyl)amino]hex-4-en-3-onato)strontium, bis(,2-dimethyl-5-[(2-methoxy-1-methylethyl)amino]hex-4-en-3-onato)strontium, bis(2,2-dimethyl-5-[(2,2-dimethoxyethyl)amino]hex-4-en-3-onato)strontium, bis(2,2-dimethyl-5-[(2,2-dimethoxyethyl)amino]hex-4-en-3-onato)barium, and bis(2,2-dimethyl-5-[(tetrahydrofuran-2-ylmethyl)amino]hex-4-en-3-onato)strontium.

9. A liquid composition suitable for use to deposit a group 2 metal-containing film on a substrate by a vapor deposition process, the composition comprising:
a polydentate β-ketoiminate according to any one of Claims 1 to 8; and
a solvent selected from: aliphatic hydrocarbons, aromatic hydrocarbons, ethers, esters, nitriles, organic esters, organic amines, polyamines, organic amides, alcohols, and mixtures thereof.

10. A vapor deposition process for forming a Group 2 metal oxide layer on a substrate, the process comprising the steps of:
dissolving a polydentate β-ketoiminate according to any one of Claims 1 to 8 in a solvent, wherein the solvent is selected from: aliphatic hydrocarbons, aromatic hydrocarbons, ethers, esters, nitriles, organic esters, organic amines, polyamines, organic amides, alcohols, and mixtures thereof; and
depositing a layer of the Group 2 metal-containing film on a substrate.

11. A method for making a metal-containing polydentate β-ketoiminate comprising reacting a polydentate β-ketoiminate ligand with a metal alkoxide in an organic solvent or a mixture of solvents.

12. The method of Claim 11 wherein the metal is selected from magnesium, calcium, strontium and barium, and wherein the polydentate β-ketoiminate ligand is selected from: 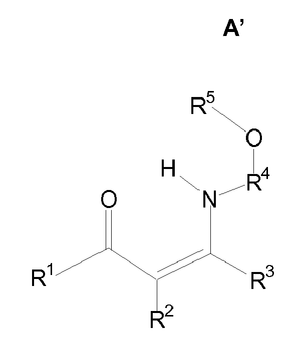 wherein:
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁴ is a C₁ to C₆ linear or branched alkylene; and
R⁵ is selected from: a C₁ to C₁₀ alkyl, a C₁ to C₁₀ fluoroalkyl, a C₃ to C₁₀ cycloaliphatic, and a C₆ to C₁₀ aryl; 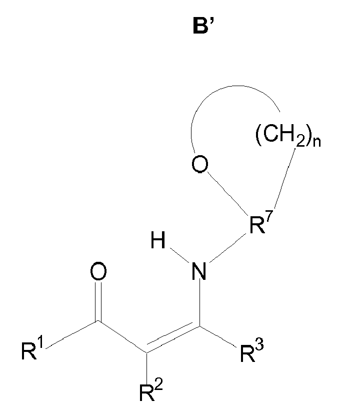
wherein:
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁷ is an organic group comprising 2 or 3 carbon atoms; and
n = 3, 4, or 5; and 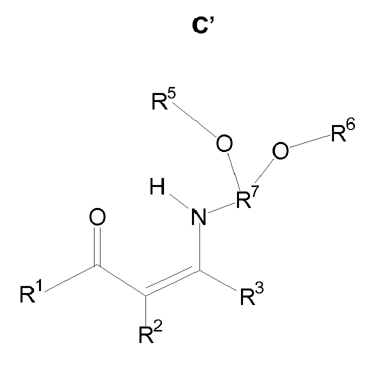
wherein:
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁷ is an organic group comprising 2 or 3 carbon atoms; and
R⁵ and R⁶ are each independently selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl.

13. A metal-containing polydentate β-ketoiminate that is the reaction product of a polydentate β-ketoiminate ligand and a metal alkoxide or metal amide, wherein the metal is selected from magnesium, calcium, strontium and barium, and the polydentate β-ketoiminate ligand is selected from: 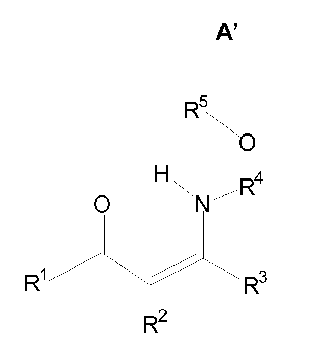 wherein:
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁴ is a C₁ to C₆ linear or branched alkylene; and
R⁵ is selected from: a C₁ to C₁₀ alkyl, a C₁ to C₁₀ fluoroalkyl, a C₃ to C₁₀ cycloaliphatic, and a C₆ to C₁₀ aryl; 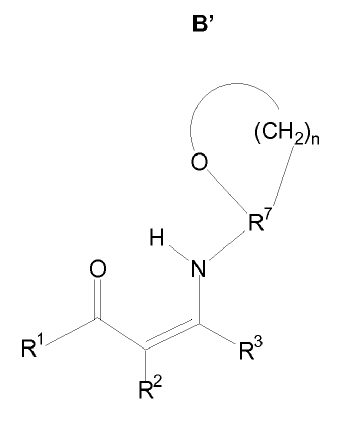
wherein:
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁷ is an organic group comprising 2 or 3 carbon atoms; and
n = 3, 4, or 5; and 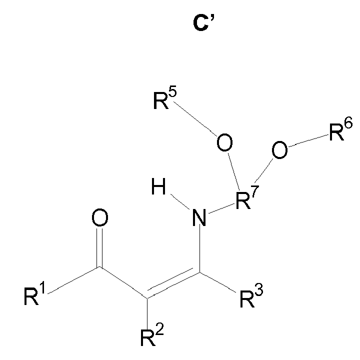
wherein:
R¹ is selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl;
R² is selected from: hydrogen, a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R³ is selected from: a C₁ to C₁₀ alkyl, a C₁ to C₁₀ alkoxyalkyl, a C₁ to C₁₀ alkoxy, a C₃ to C₁₀ cycloaliphatic, and a C₆ to C₁₀ aryl;
R⁷ is an organic group comprising 2 or 3 carbon atoms; and
R⁵ and R⁶ are each independently selected from: a C₁ to C₁₀ alkyl, a C₁ to C₁₀ alkoxyalkyl, a C₁ to C₁₀ alkoxy, a C₁ to C₁₀ fluoroalkyl, a C₃ to C₁₀ cycloaliphatic, and a C₆ to C₁₀ aryl.

14. The method or reaction product of any one of Claims 11 to 13 wherein the metal alkoxide comprises an alkoxide selected form: methoxide, ethoxide, iso-propoxide, n-propoxide, tert-butoxide, sec-butoxide, iso-butoxide, and mixtures thereof.

15. The method or reaction product of any one of Claims 11 to 14 wherein the polydentate β-ketoiminate ligand is selected from: 2,2-dimethyl-5-[(2-methoxyethyl)amino]hex-4-en-3-one, 2,2-dimethyl-5-[(2-methoxy-1-methylethyl)amino]hex-4-en-3-one, 4-[(2,2-dimethoxyethyl)amino]pent-3-en-2-one, 2,2-dimethyl-5-[(2,2-dimethoxyethyl)amino]hex-4-en-3-one, and 2,2-dimethyl-5-[(tetrahydrofuran-2-ylmethyl)amino]hex-4-en-3-one.
